# EUROPEAN PATENT APPLICATION

(11) **EP 4 663 760 A1**
(43) Date of publication of application: **17.12.2025**
(21) Application number: 24771107.0
(22) Date of filing: 06.03.2024
(51) Int. Cl.: C12N 15/77, C12N 9/06, C12P 13/06

(54) **L-ALANINE PRODUCING MICROORGANISM AND METHOD FOR PRODUCING L-ALANINE USING SAME**

(30) Priority: 10.03.2023 KR 20230032035
(71) Applicant: CJ CheilJedang Corporation, Seoul 04560 (KR)
(72) Inventor: KIM, Ju-yeon, Seoul 04560 (KR); KIM, Min-Jung, Seoul 04560 (KR); LEE, Ji Hye, Seoul 04560 (KR)
(74) Representative: Meissner Bolte Partnerschaft mbB
(86) International application number: PCT/KR2024/002903
(87) International publication number: WO 2024/191107

(57) **Abstract**

The present disclosure relates to a microorganism of the genus *Corynebacterium* having an L-alanine-producing ability, into which the activity of alanine dehydrogenase is introduced; a method for producing L-alanine, comprising culturing the microorganism in a medium; a composition for producing L-alanine, comprising the microorganism, a culture product of the microorganism, a fermented product of the microorganism, or a combination of two or more thereof; and the use of the microorganism for the production of L-alanine.

## Description

### [Technical Field]

The present disclosure relates to a microorganism of the genus *Corynebacterium* having an L-alanine-producing ability, into which the activity of alanine dehydrogenase is introduced; a method for producing L-alanine, including culturing the microorganism in a medium; a composition for producing L-alanine, including the microorganism, a culture product of the microorganism, a fermented product of the microorganism, or a combination of two or more thereof; and the use of the microorganism for the production of L-alanine.

### [Background Art]

L-alanine is an important amino acid which is widely applied in the fields of chemistry, food, medicine, *etc.* It is an amino acid that produces sweet taste and is especially used in the food industry as a flavor enhancer and nutritional supplement. As of 2020, the global market of alanine is valued at over 250 million USD, with an annual alanine production of approximately 500 tons (The Expresswire, 2020; Wendisch, 2014).

In this regard, chemical synthesis or enzyme conversion is mainly used to produce L-alanine. The chemical synthesis is achieved by modifying the Strecker synthesis or the Bucherer-Bergs reaction (Legnani et al., 2021), and the enzyme conversion is achieved by ammonium fumarate from L-aspartic acid (Takamatsu et al., 1982). Most industrial production is achieved through the enzyme conversion process. Here, L-aspartic acid, the substrate, is produced from petroleum, and the production of fumaric acid is also dependent on petroleum, which requires a high cost, and there is a problem of low yield when replacing the petroleum.

Accordingly, as an alternative, fermentation by microorganisms can supply abundant carbon sources at low cost. Microorganisms naturally produce L-alanine by aminotransferase or alanine dehydrogenase. However, there are limitations in producing L-alanine using naturally-derived strains due to low productivity.

Therefore, research for effectively increasing L-alanine-producing ability is still needed.

### [Disclosure]

### [Technical Problem]

The present inventors have confirmed that when the activity of alanine dehydrogenase is introduced into a microorganism of the genus *Corynebacterium,* the L-alanine-producing ability of the microorganism is increased compared to that of a non-modified microorganism, thereby completing the present disclosure.

### [Technical Solution]

It is one object of the present disclosure to provide a microorganism of the genus *Corynebacterium* having an L-alanine-producing ability, into which the activity of alanine dehydrogenase is introduced.

In one embodiment, the alanine dehydrogenase may be derived from *Bacillus licheniformis, Bacillus amyloliquefaciens,* or *Laceyella sacchari.*

In another embodiment, the alanine dehydrogenase may be encoded by the *ald1, ald2,* or *alaD* gene.

In still another embodiment, the alanine dehydrogenase may comprise an amino acid sequence of SEQ ID NO: 1, SEQ ID NO: 3, SEQ ID NO: 5, or SEQ ID NO: 7

In yet another embodiment, the gene encoding the alanine dehydrogenase may comprise a nucleotide sequence of SEQ ID NO: 2, SEQ ID NO: 4, SEQ ID NO: 6, or SEQ ID NO: 8.

As the microorganism according to any one of the above-described embodiments, the microorganism of the genus *Corynebacterium* may be *Corynebacterium glutamicum.*

As the microorganism according to any one of the above-described embodiments, the microorganism of the genus *Corynebacterium* may have an increased L-alanine-producing ability compared to a non-modified microorganism.

It is another object of the present disclosure to provide a method for producing L-alanine, including: culturing a microorganism of the genus *Corynebacterium* having an L-alanine-producing ability, into which the activity of alanine dehydrogenase is introduced, in a medium

In one embodiment, the alanine dehydrogenase may be derived from *Bacillus licheniformis, Bacillus amyloliquefaciens,* or *Laceyella sacchari.*

In another embodiment, the method may further comprise recovering a target substance from the cultured microorganism, a culture product of the microorganism, a fermented product of the microorganism, or the culture medium.

It is still another object of the present disclosure to provide a composition for producing L-alanine, including a microorganism of the genus *Corynebacterium* having an L-alanine-producing ability, into which the activity of alanine dehydrogenase is introduced, a culture product of the microorganism, a fermented product of the microorganism, or a combination of two or more thereof.

In one embodiment, the alanine dehydrogenase may be derived from *Bacillus licheniformis, Bacillus amyloliquefaciens,* or *Laceyella sacchari.*

It is yet another object of the present disclosure to provide the use of a microorganism of the genus *Corynebacterium,* into which the activity of alanine dehydrogenase is introduced, for the production of L-alanine.

In one embodiment, the alanine dehydrogenase may be derived from *Bacillus licheniformis, Bacillus amyloliquefaciens,* or *Laceyella sacchari.*

### [Advantageous Effects]

The microorganism of the genus *Corynebacterium* having an L-alanine-producing ability, into which the activity of alanine dehydrogenase is introduced, of the present disclosure can produce L-alanine with high yield, and thus may be effectively employed for the industrial production of L-alanine.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or".

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising/including" means the presence of features, steps or components recited after the term, and does not exclude the presence or addition of one or more features, steps or components. The components or features recited after the term "comprising/including" herein may be essential or mandatory. However, in some embodiments, the term may further include any other or non-essential components or features.

One aspect of the present disclosure provides a microorganism of the genus *Corynebacterium* having an L-alanine-producing ability, into which the activity of alanine dehydrogenase is introduced.

In one embodiment, the alanine dehydrogenase may be derived from *Bacillus licheniformis, Bacillus amyloliquefaciens,* or *Laceyella sacchari.*

The activity of the alanine dehydrogenase may be defined as an increased L-alanine-producing ability of the microorganism, into which the activity of alanine dehydrogenase is introduced, of the present disclosure, compared to the L-alanine-producing ability of a natural wild-type microorganism or a non-modified microorganism (*e.g.,* a strain before the activity of the alanine dehydrogenase of the present disclosure is introduced), but is not limited thereto.

For example, the activity of the alanine dehydrogenase may be evaluated by measuring the L-alanine-producing ability or yield, but is not limited thereto.

As used herein, the term "alanine dehydrogenase" may refer to an oxidation/reduction enzyme that catalyzes a reversible conversion of L-alanine from pyruvate with a coenzyme NAD+/NADH.

Specifically, the alanine dehydrogenase of the present disclosure may be a protein having the activity of alanine dehydrogenase encoded by the *ald1, ald2,* or *alaD* gene, but the type thereof is not particularly limited as long as the protein has activity corresponding to the activity of alanine dehydrogenase and whose activity is introduced into a microorganism of the genus *Corynebacterium,* thereby enhancing the L-alanine-producing ability. The alanine dehydrogenase encoded by the *ald1, ald2,* or *alaD* gene is known in the art, and the amino acid and polynucleotide sequences of the alanine dehydrogenase can be obtained from a known database, NCBI's GenBank. *etc.,* but is not limited thereto.

In the present disclosure, the alanine dehydrogenase may be an alanine dehydrogenase derived from *Bacillus licheniformis, Bacillus amyloliquefaciens,* or *Laceyella sacchari,* but is not limited thereto.

In one example of the present disclosure, the alanine dehydrogenase derived from *Bacillus licheniformis* may be a protein comprising the amino acid sequence of SEQ ID NO: 1 or SEQ ID NO: 3, but is not limited thereto. The alanine dehydrogenase derived from *Bacillus amyloliquefaciens* may be a protein comprising the amino acid sequence of SEQ ID NO: 5, but is not limited thereto. The alanine dehydrogenase derived from *Laceyella sacchari* may be a protein comprising the amino acid sequence of SEQ ID NO: 7, but is not limited thereto.

For example, the protein having the activity of alanine dehydrogenase in the present disclosure may be a protein comprising the amino acid sequence of alanine dehydrogenase derived from *Bacillus licheniformis* (SEQ ID NO: 1 or SEQ ID NO: 3), the amino acid sequence of alanine dehydrogenase derived from *Bacillus amyloliquefaciens* (SEQ ID NO: 5), or the the amino acid sequence of alanine dehydrogenase derived from *Laceyella sacchari* (SEQ ID NO: 7), but is not limited thereto.

In one example, the alanine dehydrogenase may comprise the amino acid sequence of SEQ ID NO: 1, SEQ ID NO: 3, SEQ ID NO: 5, or SEQ ID NO: 7, or an amino acid sequence having 60% or more homology thereto, but is not limited as long as it has an activity of alanine dehydrogenase. Specifically, a polypeptide having the activity of alanine dehydrogenase may have, comprise, consist of, or essentially consist of the amino acid sequence of SEQ ID NO: 1, SEQ ID NO: 3, SEQ ID NO: 5, or SEQ ID NO: 7; or an amino acid sequence having at least 60%, 70%, 80%, 85%, 90%, 95%, 96%, 97%, 98%, or 99% or more homology or identity to SEQ ID NO: 1, SEQ ID NO: 3, SEQ ID NO: 5, or SEQ ID NO: 7.

With respect to amino acid sequences in the present disclosure, although it is described as a polypeptide "comprising/including" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted or added may fall within the scope of the present disclosure if it has activity identical or corresponding to the protein consisting of the amino acid sequence of the corresponding sequence number. For example, sequence additions upstream or downstream of the amino acid sequences that do not alter the function of the protein, naturally occurring mutations, silent mutation thereof, or conservative substitutions are not excluded, as long as the protein has activity identical or corresponding to the activity of the modified protein, and it will be apparent that such sequence additions or mutations belong to the scope of the present disclosure.

For example, it may be a case of sequence additions that do not alter the function of the variant polypeptide of the present disclosure, naturally occurring mutations, silent mutations thereof, or conservative substitutions at the N-terminus, C-terminus and/or within the amino acid sequences. For example, the polypeptides may be conjugated with a signal (or leader) sequence at the N-terminal involved in the translocation of proteins co-translationally or post-translationally. Further, the polypeptides may also be conjugated with another sequence or linker to identify, purify, or synthesize the polypeptides.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chain (electrically charged amino acids) include arginine, lysine, histidine, glutamic acid, aspartate; and amino acids having uncharged side chains (uncharged amino acid; also referred to as neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids can be classified according to their size into 5 groups, starting from amino acid groups with a relatively small volume, *i.e.,* glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto. Typically, conservative substitutions may have little or no effect on the activity of the polypeptide.

Additionally, the nucleotide sequence encoding the alanine dehydrogenase may be a nucleotide sequence encoding a protein that exhibits the activity of alanine dehydrogenase and whose activity is introduced into a microorganism of the genus *Corynebacterium,* thereby enhancing the L-alanine-producing ability. For example, it may be a nucleotide sequence encoding the alanine dehydrogenase derived from *Bacillus licheniformis* (SEQ ID NO: 1 or SEQ ID NO: 3), the alanine dehydrogenase derived from *Bacillus amyloliquefaciens* (SEQ ID NO: 5), or the alanine dehydrogenase derived from *Laceyella sacchari* (SEQ ID NO: 7), but is not limited thereto.

For example, the L- alanine dehydrogenase having the amino acid sequence of SEQ ID NO: 1, SEQ ID NO: 3, SEQ ID NO: 5, or SEQ ID NO: 7 may be encoded by a polynucleotide that may have, comprise, consist of, or essentially consist of a nucleotide sequence of SEQ ID NO: 2, SEQ ID NO: 4, SEQ ID NO: 6, or SEQ ID NO: 8, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 2, SEQ ID NO: 4, SEQ ID NO: 6, or SEQ ID NO: 8, but is not limited thereto. In addition, the nucleotide sequence of SEQ ID NO: 2, SEQ ID NO: 4, SEQ ID NO: 6, or SEQ ID NO: 8 can be obtained from a known database, NCBI's GenBank, *etc.,* but is not limited thereto.

In the present disclosure, for example, the gene comprising the nucleotide sequence of SEQ ID NO: 2, SEQ ID NO: 4, SEQ ID NO: 6, or SEQ ID NO: 8 may be used interchangeably with a polynucleotide comprising the nucleotide sequence of SEQ ID NO: 2, SEQ ID NO: 4, SEQ ID NO: 6, or SEQ ID NO: 8, a gene or polynucleotide having the nucleotide sequence of SEQ ID NO: 2, SEQ ID NO: 4, SEQ ID NO: 6, or SEQ ID NO: 8, and a gene or polynucleotide consisting of the nucleotide sequence of SEQ ID NO: 2, SEQ ID NO: 4, SEQ ID NO: 6, or SEQ ID NO: 8.

The polynucleotide of the present disclosure may undergo various modifications in the coding region without changing the amino acid sequence of the alanine dehydrogenase of the present disclosure, due to codon degeneracy or in consideration of the codons preferred in an organism in which the alanine dehydrogenase of the present disclosure is to be expressed. Therefore, based on codon degeneracy, it is apparent that polynucleotides which may be translated into polypeptides consisting of the amino acid sequence of the alanine dehydrogenase of the present disclosure or polypeptides having a homology or identity thereto may also be included. For example, the polynucleotide of the present disclosure may be SEQ ID NO: 2, SEQ ID NO: 4, SEQ ID NO: 6, or SEQ ID NO: 8; or a degenerated sequence thereof.

Additionally, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions to encode the alanine dehydrogenase of the present disclosure without limitation.

As used herein, the term "homology" or "identity" refers to the degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used together with a default gap penalty established by the program being used. Substantially, homologous or identical sequences are generally expected to hybridize to all or at least about 50%, 60%, 70%, 80% or 90% of the entire length of the sequences under moderate or highly stringent conditions. Polynucleotides that contain degenerate codons instead of codons in the hybridizing polynucleotides may also be considered.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et at, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (*i.e.,* nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be determined by comparing sequences via Southern hybridization experiments under defined stringent conditions, and the appropriate hybridization conditions to be defined may be determined by way of a method within the scope of the present disclosure, which is known to those skilled in the art (*e.g.,* J. Sambrook et al., Molecular Cloning, A Laboratory Manual, 2nd Edition, Cold Spring Harbor Laboratory press, Cold Spring Harbor, N.Y., 1989; F. M. Ausubel et al., Current Protocols in Molecular Biology, John Wiley & Sons, Inc., New York), but is not limited thereto.

As used herein, the "stringent conditions" refer to conditions which allow the specific hybridization between polynucleotides. Such conditions are disclosed in detail in the literature (*e.g.,* J. Sambrook et al., supra). For example, the stringent conditions may include conditions under which polynucleotides having high homology or identity, polynucleotides having 60% or more, 70% or more, 75% or more, 80% or more, 85% or more, 90% or more, 95% or more, 96% or more, 97% or more, 98% or more, or 99% or more hybridize with each other, while polynucleotides having homology or identity lower than the above homology or identity do not hybridize with each other; or may comprise ordinary washing conditions of Southern hybridization, *i.e.,* washing once, specifically twice or three times, at a salt concentration and a temperature corresponding to 60° C., 1×SSC, 0.1% SDS, specifically 60° C., 0.1×SSC, 0.1% SDS, and more specifically 68° C., 0.1×SSC, 0.1% SSD.

Hybridization requires that two nucleotides have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the present disclosure may also comprise an isolated nucleic acid fragment complementary to the entire sequence as well as a base sequence substantially similar thereto.

For example, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C or 65°C, but is not limited thereto, and may be appropriately adjusted by those skilled in the art depending on the purpose thereof.

The appropriate stringency for hybridizing polynucleotides depends on the length and degree of complementarity of the polynucleotides, and these variables are well known in the art (*see* Sambrook et al., supra, 9.50-9.51, 11.7-11.8).

As used herein, the term "L-alanine" refers to an L-amino acid with the chemical formula of HO₂CCH(NH₂)CH₃, which is one of the essential amino acids.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc*., and may be a microorganism including genetic modification to produce a desired polypeptide, protein or product. In the present disclosure, the "microorganism" and "strain" have the same meaning, and may be used interchangeably with each other without limitation.

For example, the microorganism of the present disclosure may be a microorganism into which the activity of alanine dehydrogenase is introduced (*e.g.,* a recombinant strain), but is not limited thereto.

As used herein, the term "microorganism having an L-alanine-producing ability", which is a prokaryotic or eukaryotic microbial strain capable of producing L-alanine in an organism, may comprise all of microorganisms, in which an L-alanine-producing ability has been imparted to a parent strain having no L-alanine-producing ability, or microorganisms that endogenously have an L-alanine-producing ability. The L-alanine-producing ability may be imparted or enhanced by improvement of species.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. The "non-modified microorganism" may be used interchangeably with "strain before modification", "microorganism before modification", "non-mutant strain", "non-modified strain", "non-mutant microorganism", "parent strain before mutation", "wild-type microorganism", "reference microorganism", or "standard microorganism". In the present disclosure, the non-modified microorganism may refer to a strain into which the activity of alanine dehydrogenase is not introduced, or a strain before the introduction of alanine dehydrogenase, but is not limited thereto. In addition, in the present disclosure, the non-modified microorganism may be a microorganism which does not comprise the amino acid sequence of SEQ ID NO: 1, SEQ ID NO: 3, SEQ ID NO: 5, or SEQ ID NO: 7, or the nucleotide sequence of SEQ ID NO: 2, SEQ ID NO: 4, SEQ ID NO: 6, or SEQ ID NO: 8, but is not limited thereto.

For the purpose of the present disclosure, the microorganism of the present disclosure may include all microorganisms capable of producing the desired L-alanine by introducing the activity of L alanine dehydrogenase. For example, the microorganism of the present disclosure is characterized in that the activity of alanine dehydrogenase is introduced, thereby increasing the L-alanine-producing ability, and may be a genetically modified microorganism or a recombinant microorganism, but is not limited thereto. Specifically, the recombinant strain with an increased L-alanine-producing ability may be a microorganism having an increased L-alanine-producing ability compared to a natural wild-type microorganism or a non-modified microorganism having an endogenous activity of alanine dehydrogenase, but is not limited thereto.

In one example, the microorganism having an L-alanine-producing ability, which is a prokaryotic or eukaryotic microbial strain capable of producing L-alanine in an organism, may include all of microorganisms that endogenously have an L-alanine-producing ability, or microorganisms, in which an L-alanine-producing ability has been imparted to a parent strain having no L-alanine-producing ability by the activity of the alanine dehydrogenase introduced in the present disclosure. The L-alanine-producing ability may be imparted or enhanced by improvement of species.

The microorganism of the present disclosure may include all microorganisms into which the activity of alanine dehydrogenase is introduced by various known methods.

As used herein, the term "introduction" of activity means that a gene not originally possessed by a microorganism is expressed in the microorganism, and thus the microorganism exhibits the activity of a particular protein, or the activity of a polypeptide is enhanced, increased or improved compared to the endogenous activity of the corresponding protein or the activity before modification. For example, the term may indicate that a polynucleotide encoding a particular protein is introduced into the chromosome of a microorganism, or a vector containing a polynucleotide encoding a particular protein is introduced into a microorganism, thereby exhibiting the activity of the particular protein.

The "endogenous activity" refers to the activity of a particular polypeptide originally possessed by a parent strain before transformation or a non-modified microorganism when a trait is altered by genetic variation due to a natural or artificial factor. The endogenous activity may also be interchangeably used with "activity before modification".

As used herein, the term "enhancement" of polypeptide activity means that the activity of a polypeptide is increased, as compared with the endogenous activity thereof. The enhancement may be used interchangeably with terms such as activation, up-regulation, overexpression, increase, *etc.*

In particular, the activation, enhancement, up-regulation, overexpression, increase may include both cases in which an activity not originally possessed is exhibited, or an activity is enhanced compared to the endogenous activity or the activity before modification. The enhancement", "up-regulation", "overexpression" or "increase" in the activity of a polypeptide as compared to the endogenous activity means that the activity of the polypeptide is enhanced, as compared with the activity and/or concentration (expression level) of a particular polypeptide originally possessed by a parent strain before transformation or a non-modified microorganism. For example, the term "activity not originally possessed is exhibited" may refer to the "introduction of a protein", but is not limited thereto.

The enhancement in the activity of the polypeptide, as compared with the endogenous activity, means that the activity of the polypeptide is enhanced, as compared with the activity and/or concentration (expression level) of a particular polypeptide originally possessed by a parent strain before transformation or a non-modified microorganism.

In one example, the enhancement may mean that the activity of a corresponding protein not originally possessed is exhibited, or the activity or concentration thereof is enhanced generally by about 1 %, about 10 %, about 25 %, about 50 %, about 75 %, about 100 %, about 150 %, about 200 %, about 300 %, about 400 % or about 500 %, maximum about 1000 % or about 2000 % or more, based on the activity or concentration of a wild-type protein or an initial microbial strain, but is not limited thereto.

The enhancement of the activity of the polypeptide may be achieved by introducing a foreign polypeptide or enhancing the activity of an endogenous polypeptide. Whether or not the activity of the polypeptide is enhanced may be confirmed from the activity level of the corresponding polypeptide, expression level thereof, or the increase in the amount of products produced from the corresponding polypeptide. As used herein, the term "about" refers to a range including all of ± 0.5, ± 0.4, ± 0.3, ± 0.2, ± 0.1, *etc.,* and it includes all of the values equivalent to those which come immediately after the term "about" or those in a similar range, but is not limited thereto.

The enhancement may be achieved by introducing a foreign polypeptide or enhancing the activity and/or concentration (expression level) of an endogenous polypeptide. Whether or not the activity of the polypeptide is enhanced may be confirmed from the activity level of the corresponding polypeptide, expression level thereof, or the increase in the amount of products produced from the corresponding polypeptide.

The enhancement of the activity of the polypeptide may be applied by various methods well known in the art, and is not limited as long as it can enhance the activity of a target polypeptide compared to that of the microorganism before modification. Specifically, genetic engineering and/or protein engineering well known to those skilled in the art, which is a common method of molecular biology, may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

Specifically, the enhancement of the polypeptide of the present disclosure may be achieved by:
1) increasing the intracellular copy number of a polynucleotide encoding the polypeptide;
2) modifying the expression regulatory region of a gene encoding the polypeptide on the chromosome (*e.g.,* inducing a modification within the expression regulatory region, replacing with a sequence having a stronger activity, or inserting a sequence having a stronger activity);
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g.,* modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of a polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration (expression level) of the corresponding polypeptide is increased relative to the activity or concentration of a polypeptide expressed in a wild-type strain or a microorganism before modification, or that the amount of products produced from the corresponding polypeptide is increased, but is not limited thereto.

In one example, the recombinant microorganism having an L-alanine-producing ability may include all microorganisms, which may be transformed through a vector and thus capable of producing L-alanine by introducing a foreign gene encoding the alanine dehydrogenase of the present disclosure, specifically, a foreign gene encoding the alanine dehydrogenase derived from *Bacillus licheniformis, Bacillus amyloliquefaciens,* or *Laceyella sacchari.*

For the purpose of the present disclosure, the microorganism may be one in which the activity of activity of alanine is enhanced compared to the endogenous activity by including an expression vector for expressing the foreign polynucleotide in a host cell, but is not limited thereto.

The vector of the present disclosure may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL, pSK, pSKH and pET, *etc.* may be used. Specifically, pDZ, pDC, pDCM2, pACYC177, pACYC184, pCL, pSK, pSKH130, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

As used herein, the term "operably linked" refers to a constitution of placing a regulatory sequence to an appropriate position to regulate expression of a coding sequence. Thus, the term "operably linked" includes an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, it may mean that the polynucleotide sequence is functionally linked to a promoter sequence that initiates and mediates transcription of the polynucleotide encoding the target variant polypeptide of the present disclosure.

As used herein, the term "expression" includes any step involved in the production of a polypeptide, *e.g.,* transcription, post-transcriptional modification, translation, post-translational modification, and secretion, *etc.,* but is not limited thereto.

As used herein, the term "expression vector" refers to a linear or circular nucleic acid molecule including a coding sequence and an operably linked regulatory sequence for expression thereof.

As used herein, the term "regulatory sequence" refers to a polynucleotide sequence necessary for the expression of a coding sequence. Each regulatory sequence may be native (derived from the same origin) or foreign (derived from different genes) to the coding sequence. Examples of the regulatory sequence may include a leader sequence, a polyadenylation sequence, a pro-peptide sequence, a promoter, a signal peptide sequence, an operator sequence, a sequence encoding a ribosome-binding site, and a sequence for regulating the termination of transcription and translation. The minimum units of the regulatory sequence may include a promoter, and a sequence for terminating transcription and translation.

As used herein, the term "recombinant" with respect to a cell, polynucleotide, polypeptide, or vector, indicates that the cell, polynucleotide, polypeptide or vector has been modified by the introduction of a heterologous nucleic acid or polypeptide, or the alteration of a native polynucleotide or polypeptide, or that the cell is derived from a cell thus modified. Therefore, for example, recombinant cells may express genes that are not found within the native (non-recombinant) form of the cells, or may express native genes that are otherwise abnormally expressed, under expressed or not expressed at all.

For example, the microorganism for producing L-alanine may be a microorganism into which a sequence encoding a protein consisting of the amino acid sequence of SEQ ID NO: 1, SEQ ID NO: 3, SEQ ID NO: 5, or SEQ ID NO: 7, or a protein consisting of an amino acid sequence having at least 60%, 70%, 75%, 80%, 85%, 90%, 95%, 96%, 97%, 98%, 99%, 99.5%, 99.7% or 99.9% or more homology or identity to SEQ ID NO: 1, SEQ ID NO: 3, SEQ ID NO: 5, or SEQ ID NO: 7 is introduced.

For example, the microorganism for producing L-alanine may be a microorganism endogenously comprising a nucleotide sequence capable of encoding a protein comprising an amino acid sequence having at least 80% homology to SEQ ID NO: 1, SEQ ID NO: 3, SEQ ID NO: 5, or SEQ ID NO: 7; the nucleotide sequence of SEQ ID NO: 2, SEQ ID NO: 4, SEQ ID NO: 6, or SEQ ID NO: 8, and a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, SEQ ID NO: 4, SEQ ID NO: 6, or SEQ ID NO: 8.

In one example, the microorganism with an increased L-alanine-producing ability of the present disclosure may be a microorganism with an increased L-alanine-producing ability compared to a non-modified microorganism, but is not limited thereto. In one example, the non-modified microorganism that is the target strain for comparing the increase in the L-alanine-producing ability may be ATCC13869 strain, but is not limited thereto.

In one example, the microorganism with an increased L-alanine-producing ability may have an increased L-alanine-producing ability by about 150% or more, specifically about 150% or more, about 200% or more, about 250% or more, about 260% or more, about 270% or more, about 280% or more, about 290% or more, about 300% or more, about 310% or more, about 320% or more, about 325% or more, about 326% or more, about 327% or more, about 328% or more, about 329% or more, about 330% or more, about 331% or more, about 332% or more, about 333% or more, about 334% or more, about 335% or more, or about 336% or more (the upper limit is not particularly limited, for example, about 1000% or less, about 500% or less, about 400% or less, about 390% or less, about 380% or less, about 370% or less, about 360% or less, about 350% or less, or about 340% or less), as compared to the L-alanine-producing ability of a parent strain before modification or a non-modified microorganism, but is not limited thereto, as long as it has an increased + value compared to the production ability of a parent strain before modification or non-modified microorganism. In another example, the microorganism with an increased L-alanine-producing ability may have an increased L-alanine-producing ability by about 1.5 times or more, about 2 times or more, about 2.5 times or more, about 2.6 times or more, about 2.7 times or more, about 2.8 times or more, about 2.9 times or more, about 3 times or more, about 3.1 times or more, about 3.2 times or more, 3.26 times or more, about 3.3 times or more, or about 3.36 times or more (the upper limit is not particularly limited, for example, about 10 times or less, about 5 times or less, about 4 times or less, about 3.7 times or less, about 3.5 times or less, or about 3.4 times or less) as compared to the L-alanine-producing ability of a parent strain before modification or a non-modified microorganism, but is not limited thereto.

In one example, the microorganism having an L-alanine-producing ability may be a prokaryotic cell or eukaryotic cell, but may specifically be a prokaryotic cell. The prokaryotic cell may include, for example, a microbial strain belonging to the genus *Escherichia,* the genus *Erwinia,* the genus *Serratia,* the genus *Providencia,* the genus *Corynebacterium,* the genus *Pseudomonas,* the genus *Leptospira,* the genus *Salmonella,* the genus *Brevibacteria,* the genus *Hypomononas,* the genus *Chromobacterium,* and the genus *Norcardia,* or fungi or yeasts. Specifically, the microorganism may be a microbial strain belonging to the genus *Escherichia,* the genus *Corynebacterium,* the genus *Leptospira,* and yeasts. More specifically, it may be a microbial strain belonging to the genus *Corynebacterium.*

As the microorganism according to any one of the above-described embodiments, the microorganism of the present disclosure may be a microorganism of the genus *Corynebacterium.*

In one example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* more specifically, *Corynebacterium glutamicum* or *Corynebacterium stationis,* but is not limited thereto.

Meanwhile, although it has been already known that microorganisms of the genus *Corynebacterium* can produce L-alanine, they have a significantly low L-alanine-producing ability, and the gene acting on the production mechanism or the principle of the mechanism has not been known. Accordingly, the microorganism of the genus *Corynebacterium* having an L-alanine-producing ability of the present disclosure may include all of the following: a wild-type microorganism of the genus *Corynebacterium* itself; a microorganism of the genus *Corynebacterium* in which the activity of the gene associated with the mechanism of L-alanine production is enhanced or inactivated, thus having an L-alanine-producing ability; or a microorganism of the genus *Corynebacterium* in which the activity of a foreign gene is introduced or enhanced, thus having an L-alanine-producing ability.

The microorganism having an L-alanine-producing ability of the present disclosure may be a microorganism with an enhanced L-alanine-producing ability by further enhancing the activity of the introduced alanine dehydrogenase, but is not limited thereto.

The microorganism of the present disclosure may include all microorganisms in which the activity of the introduced alanine dehydrogenase is further enhanced by by various methods in the art.

Another aspect of the present disclosure provides a method for producing L-alanine, comprising: culturing the microorganism of the genus *Corynebacterium* having an L-alanine-producing ability, into which the activity of alanine dehydrogenase is introduced, of the present disclosure in a medium.

In one embodiment, the alanine dehydrogenase may be derived from *Bacillus licheniformis, Bacillus amyloliquefaciens,* or *Laceyella sacchari.*

As used herein, the term "cultivation" means that the strain of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the strain of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.* For example, the culture medium for the microorganisms of the genus *Corynebacterium* can be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, *etc.;* sugar alcohols, such as mannitol, sorbitol, *etc*.; organic acids, such as pyruvic acid, lactic acid, citric acid, *etc.;* amino acids, such as glutamic acid, methionine, lysine, *etc.* Additionally, the carbon source may comprise natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

The nitrogen source may comprise inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may comprise monopotassium phosphate, dipotassium phosphate, or corresponding sodium-containing salts, *etc.* Examples of the inorganic compounds may comprise sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 27°C to 37°C, specifically from 30°C to 33°C, and the cultivation may be continued for 20 hours to 120 hours, but is not limited thereto.

As used herein, the term "culture product" means a culture solution, a concentrated culture solution, a dried product of a culture solution, a culture filtrate, a concentrated culture filtrate, or a dried product of a culture filtrate obtained by culturing a specific microorganism in a culture medium, and means that the culture solution may include the specific microorganism while the culture filtrate does not substantially include the specific microorganism (in particular, it substantially means excluding a specific microorganism isolated by filtration, *etc.,* but does not mean that the microorganism is completely excluded from the filtrate). The formulation of the culture product is not limited, and may be, for example, a liquid, emulsion, or solid. Specifically, for the purpose of the present disclosure, the culture product may comprise L-alanine.

As used herein, the term "fermentation" means that microorganisms are not putrefactive during the process of decomposing organic matter using their own enzymes. Fermentation reaction and decay reaction proceed by similar processes, but when decomposition produces useful substances, it is called fermentation, and when odorous or harmful substances are produced, it is called decay.

In the present disclosure, the method for obtaining a fermented product from the strain is not particularly limited, and may be obtained according to a method commonly used in the art or similar fields.

As used herein, the term "fermented product" may include not only the fermented material itself, but also all kinds of materials including fermented products produced from the strain, such as a culture medium of the strain in which the strain and the culture coexist; a fermented product produced from the culture medium; a fermented product obtained by filtering the strain from the culture medium; a fermented product obtained by sterilizing the strain from the culture medium and filtering the same, an extract obtained by extracting the fermented product or the culture medium containing the same; a diluted solution obtained by diluting the fermented product or an extract thereof; a concentrated solution; a dried product obtained by drying the fermented product or an extract thereof; and a lysate obtained by collecting and lysing the cells of the strain, *etc*.

In the method of the present disclosure, the cultivation of the microorganism may be performed by any culture conditions and culture methods known in the art. Such a cultivation process can be easily adjusted and used by those skilled in the art according to the selected strain.

The L-alanine produced by the cultivation of the present disclosure may be released into the medium or remain in the cells.

In one embodiment, the method for producing L-alanine of the present disclosure may further comprise a step of preparing the microorganism of the present disclosure, a step of preparing a medium for culturing the strain, or a combination thereof (regardless of the order, in any order), for example, prior to the culturing step.

The method for producing L-alanine of the present disclosure may further comprise a step of recovering the desired substances, specifically, L-alanine, from the cultured microorganism, a culture product of the microorganism, a fermented product of the microorganism, or the culture medium. The recovering step may be further comprised after the culturing step.

In the recovering step, the desired L-alanine may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired substances, specifically, L-alanine, can be recovered from the medium or the microorganisms using suitable methods known in the art.

Further, the method for producing L-alanine of the present disclosure may further comprise a purification step, which may be performed using an appropriate method known in the art. In one example, when the method for producing L-alanine of the present disclosure comprises both a recovering step and a purification step, the recovering step and the purification step may be performed continuously or intermittently regardless of the order or simultaneously, or may be integrated into one step, but the method is not limited thereto.

In the method of the present disclosure, the alanine dehydrogenase, introduction of activity, and L-alanine *etc.* are as described in other aspects above.

Still another aspect of the present disclosure provides a composition for producing L-alanine, comprising the microorganism of the genus *Corynebacterium* having an L-alanine-producing ability, into which the activity of alanine dehydrogenase is introduced, of the present disclosure, a culture product of the microorganism, a fermented product of the microorganism, or a combination of two or more thereof.

In one embodiment, the alanine dehydrogenase may be derived from *Bacillus licheniformis, Bacillus amyloliquefaciens,* or *Laceyella sacchari.*

The composition of the present disclosure may further comprise any suitable excipient commonly used in compositions for producing L-alanine, and such excipients may comprise, for example, preservatives, wetting agents, dispersing agents, suspending agents, buffers, stabilizers, or isotonic agents, *etc.,* but are not limited thereto.

In one embodiment, each component present in the composition of the present disclosure may be contained in a microbiologically effective amount, or in an amount that can be appropriately present in the composition for production.

In the composition of the present disclosure, the alanine dehydrogenase, introduction of activity, and L-alanine *etc.* are as described in other aspects above.

Yet another aspect of the present disclosure provides the use of the microorganism of the genus *Corynebacterium* having an L-alanine-producing ability, into which the activity of alanine dehydrogenase is introduced, of the present disclosure for the production of L-alanine.

In one embodiment, the alanine dehydrogenase may be derived from *Bacillus licheniformis, Bacillus amyloliquefaciens,* or *Laceyella sacchari.*

In the use of the present disclosure, the alanine dehydrogenase, introduction of activity, and L-alanine *etc*. are as described in other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Evaluation of L-alanine-Producing Ability of L-alanine-Producing Microorganisms Introduced with Exogenous Alanine Dehydrogenase -1

### Example 1-1: Construction of Vectors for Introducing Exogenous Alanine Dehydrogenase Gene

In order to construct strains introduced with an exogenous alanine dehydrogenase into *Corynebacterium glutamicum* ATCC13869, vectors were constructed to introduce genes encoding four types of alanine dehydrogenase.

Specifically, vectors containing respective genes linked to the Pcj7 promoter were constructed.

First, an intergenic region was selected as the site for copy insertion, and the sequences of restriction enzymes Xbal and Xhol (tctagactcgag) were inserted at the downstream region of NCgl2195, and homologous regions of 1.0 kb were inserted on each side. Thereafter, the chromosomal gene of *Corynebacterium glutamicum* ATCC13869 strain was isolated using a G-spin total DNA extraction mini kit (Cat. No. 17045, Intron) according to the protocols provided in the kit, and PCR was performed using a primer pair of SEQ ID NOS: 22 and 23 and a primer pair of SEQ ID NOS: 24 and 25 to obtain gene fragments (NCgl2195down_A, NCgl2195down_B), respectively. PCR was performed under conditions of denaturation at 95°C for 5 minutes, followed by 30 cycles of denaturation at 95°C for 30 seconds, annealing at 55°C for 30 seconds, and polymerization at 72°C for 30 seconds, and then polymerization at 72°C for 5 minutes. The two gene fragments, which were cleaved with BamHI and Sall restriction enzymes, were ligated to linear pDCM2 (Korean Publication No. 10-2020-0136813) using T4 ligase (New England Biolabs, Beverly, MA). The resulting vector was named pDCM2-ΔNCgl2195down.

The four types of chromosomal genes of *Bacillus licheniformis-derived ald1* gene (SEQ ID NO: 2), *Bacillus licheniformis*-derived *ald2* gene (SEQ ID NO: 4), *Bacillus amyloliquefaciens*-derived *alaD* gene (SEQ ID NO: 6), or *Laceyella sacchari-*derived *ald2* gene (SEQ ID NO: 8) were each obtained using a G-spin total DNA extraction mini kit (Cat. No. 17045, Intron).

Specifically, the genes were isolated according to the protocols provided in the mini kit, and PCR was performed using primer pairs of SEQ ID NOS: 9 and 10, and SEQ ID NOS: 11 and 12, primer pairs of SEQ ID NOS: 9 and 13, and SEQ ID NOS: 14 and 15, primer pairs of SEQ ID NOS: 9 and 16, and SEQ ID NOS: 17 and 18, and primer pairs of SEQ ID NOS: 9 and 19, and SEQ ID NOS: 20 and 21 for respective genes to obtain four Pcj7 promoter fragments that can be ligated to each of the four gene fragments, and four gene fragments ligated thereto (*ald1*(*B.li*), *ald2*(*B.li*), *ald2*(*L.sa*), *alaD*(*B.am*)), respectively. PCR was performed under conditions of denaturation at 95°C for 5 minutes, followed by 30 cycles of denaturation at 95°C for 30 seconds, annealing at 55°C for 30 seconds, and polymerization at 72°C for 30 seconds, and then polymerization at 72°C for 5 minutes. As a result, four 364 bp of Pcj7 fragments and polynucleotides of respective genes (1,276 bp of *ald1*(*B.li*), 1,283 bp of *ald2*(*B.li*), 1,271 bp of *alaD*(*B.am*), and 1,274bp of *ald2*(*L.sa*)) were obtained.

The two gene fragments (Pcj7 and respective genes), which were cleaved with Xbal and Xhol restriction enzymes, were ligated to linear pDCM2-ΔNCgl2195down using T4 ligase (New England Biolabs, Beverly, MA). The resulting vectors were named pDCM2-ΔNCgl2195down::Pcj7_ald1(B.li), pDCM2-ΔNCgl2195down::Pcj7_ald2(B.li), pDCM2-ΔNCgl2195down::Pcj7_alaD(B.am), pDCM2-ΔNCgl2195down::Pcj7_ald2(L.sa), respectively.

**[Table 1]**

| SEQ ID NO: | Name | Sequence |
|---|---|---|
| 9 | Pcj7_F_N2195dow n | GAGATAGAGGTCTTTACGtctagaagaaacatccca gcgc |
| 10 | Pcj7_R_ald1(B.li) | CTTTAGGAATTCCAATGATCATgagtgtttcctttcgt tggg |
| 11 | ald1(B.li)_F_Pcj7 | gtacccaacgaaaggaaacactcATGATCATTGGAAT TCCTAAAG |
| 12 | ald(B.li)_R_N2195 down | GTGAAGGAAAGAGTTCctcgagTTAAAGCAGA ATGGAAAAAGG |
| 13 | Pcj7_R_ald2(B.li) | CTTTCGGTACGCCGATAATCATgagtgtttcctttcg ttg |
| 14 | ald2(B.li)_F_Pcj7 | gtacccaacgaaaggaaacactcATGATTATCGGCGT ACCG |
| 15 | ald2(B.li)_R_N219 5down | GAAGGAAAGAGTTCctcgagCTGCAATTGCGG CAAAATTG |
| 16 | Pcj7_R_alaD(B.am ) | GAACCCCGATAATCATgagtgtttcctttcgttg |
| 17 | alaD(B.am)_F_Pcj 7 | caacgaaaggaaacactcATGATTATCGGGGTTC |
| 18 | alaD(B.am)_R_N2 195down | GTGAAGGAAAGAGTTCctcgagTCCGGGATAT GAATTGATATTC |
| 19 | Pcj7_R_ald2(L.sa) | CTGTTGGCACTCCTACTTTCATgagtgtttcctttcgt tgggtac |
| 20 | ald2(L.sa)_F_Pcj7 | gtacccaacgaaaggaaacactcATGAAAGTAGGAG TGCCAACAG |
| 21 | ald2(L.sa)_R_N21 95down | GAAGGAAAGAGTTCctcgagGCTCCATCGTTT CAAACG |
| 22 | N2195down_AF | ctcggtacccggggatccTTCCCATGGAAACTCTCG |
| 23 | N2195down_AR | GTGTGAAGGAAAGAGTTCctcgagtctagaCGTA AAGACCTCTATC |
| 24 | N2195down_BF | GAGATAGAGGTCTTTACGtctagactcgagGAAC TCTTTCCTTCAC |
| 25 | N2195down_BR | catgcctgcaggtcgacTTGATGAGCAGCAGTGG |

### Example 1-2: Construction of Microorganisms Introduced with Exogenous Alanine Dehydrogenase

The four types of vectors pDCM2-ΔNCgl2195down::Pcj7_ald1(B.li), pDCM2-ΔNCgl2195down::Pcj7_ald2(B.li), pDCM2-ΔNCgl2195down::Pcj7_alaD(B.am), pDCM2-ΔNCgl2195down::Pcj7_ald2(L.sa) constructed in Example 1-1 were each transformed into *Corynebacterium glutamicum* ATCC13869, the parent strain, by electroporation, and then the strains, in which the variant gene and the vector were inserted into the chromosome, were selected as primary candidates in a selection medium containing 25 mg/L of kanamycin. Thereafter, the strains were inserted into the endogenous ΔNCgl2195down intergenic region on the chromosome through a secondary crossover process using homology between the gene existing on the chromosome and the gene being inserted through the vector. Final strains, from which the vector containing a kanamycin resistance gene was removed, were obtained. The resulting strains were primarily examined through PCR using a primer pair of SEQ ID NO: 26 and SEQ ID NO: 27, and were finally confirmed through sequencing. The thus-obtained strains introduced with Pcj7_ald1(B.li), Pcj7_ald2(B.li), Pcj7_alaD(B.am), and Pcj7_ald2(L.sa) were named CJ0183, CJ0184, CJ0192, and CJ0185, respectively.

**[Table 2]**

| SEQ ID NO: | Name | Sequence |
|---|---|---|
| 26 | N2195down_CF | CACCTGGGATGGCAGCAAACTTGAAC TTG |
| 27 | N2195down_CR | CAGGATGTTTGGGCTGGTGAATCATAG |

### Example 1-3: Evaluation of L-alanine-Producing Ability of L-alanine-Producing Microorganisms Introduced with Exogenous Alanine Dehydrogenase

In order to confirm the L-alanine productivity of the *Corynebacterium glutamicum* CJ0183, CJ0184, CJ0192, and CJ0185 constructed in Example 1-2 above, the strains were cultured as follows:
The *Corynebacterium glutamicum* ATCC13869 and the four types of variant strains were inoculated into a 250-mL corner-baffled flask containing 25 mL of the following seed medium and was cultured with shaking at 200 rpm at 30°C for 20 hours to obtain a seed culture solution. Thereafter, 1 mL of the seed culture solution was inoculated into a 250-mL corner-baffled flask containing 24 mL of the following production medium and was cultured with shaking at 200 rpm at 30°C for 48 hours to prepare L-alanine.

After completion of the culture, the production of L-alanine was measured using liquid high-performance chromatography (Agilent, 1260 Infinity LC system), and the production of L-alanine in the culture solution for each strain tested was measured. The compositions of the seed medium and production medium are as follows, and the L-alanine concentrations in the culture solution for each strain tested are shown in Table 3 below.

### <Seed Medium>

Glucose (anhydrous glucose) 20g/L, Polypeptone 10g/L, Yeast extract 10g/L, (NH₄)₂SO₄ 10g/L, Urea 1.5g/L, KH₂PO₄ 5.2g/L, K₂HPO₄ 10.7g/L, d-Biotin 1.8mg/L, Thiamine-HCl 9mg/L, CAPA 9mg/L, NCA 60mg/L, MGSO₄ 0.5g/L

### <Production Medium>

CaCO₃ 30g/L, Sucrose 57g/L, BM 6g/L, MgSO₄ 0.5g/L, (NH₄)₂SO₄ 50g/L, KH₂PO₄ 1g/L, Yeast extract 2g/L, Ammonium acetate 6.28g/L, d-Biotin 0.05mg/L, Thiamine-HCl 0.1mg/L, MnSO₄ 6.7mg/L, FeSO₄ 10mg/L

**[Table 3]**

| | ATCC13869 (Parent strain) | CJ0183 | CJ0184 | CJ0192 | CJ0185 |
|---|---|---|---|---|---|
| L-alanine Concentratio n(g/L) | 6.1 | 20.5 | 19.9 | 18.9 | 15.2 |

As a result, as shown in Table 3, the *Corynebacterium glutamicum* ATCC13869, the parent strain, produced L-alanine at a concentration of 6.1 g/L, but the exogenous alanine dehydrogenase-introduced strains CJ0183, CJ0184, CJ0192, and CJ0185 according to the present disclosure produced L-alanine at concentrations of 20.5 g/L, 19.9 g/L, 18.9 g/L, and 15.2 g/L, respectively, confirming that the L-alanine productivity was increased by 336%, 326%, 310%, and 250% compared to the parent strain.

Accordingly, it was confirmed that L-alanine could be produced at high concentrations in the microorganism of the genus *Corynebacterium* by introducing the alanine dehydrogenase of the present disclosure.

### Example 2: Evaluation of L-alanine-Producing Ability of L-alanine-Producing Microorganisms Introduced with Exogenous Alanine Dehydrogenase -2

### Example 2-1: Construction of Recombinant Vectors with Additional Copies of Exogenous Alanine Dehydrogenase Gene

In order to construct strains in which one additional copy of the exogenous alanine dehydrogenase was introduced into the CJ0183 and CJ0184 strains constructed in Example 1-2, one additional copy of the gene encoding alanine dehydrogenase of *Bacillus licheniformis*-derived *ald1* gene (SEQ ID NO: 2) was introduced in a form linked to the Pcj7 promoter.

First, an intergenic region was selected as the site for copy insertion, and the sequences of restriction enzymes Notl and Xhol (gcggccgcctcgag) were inserted at the downstream region of NCgl1292, and homologous regions of 1.0 kb were inserted on each side. Thereafter, the chromosomal gene of *Corynebacterium glutamicum* ATCC13869 strain was isolated using a G-spin total DNA extraction mini kit (Cat. No. 17045, Intron) according to the protocols provided in the kit, and PCR was performed using a primer pair of SEQ ID NOS: 28 and 29 and a primer pair of SEQ ID NOS: 30 and 31 to obtain gene fragments (NCgl1292down_A, NCgl1292down_B), respectively. PCR was performed under conditions of denaturation at 95°C for 5 minutes, followed by 30 cycles of denaturation at 95°C for 30 seconds, annealing at 55°C for 30 seconds, and polymerization at 72°C for 30 seconds, and then polymerization at 72°C for 5 minutes. The two gene fragments, which were cleaved with BamHI and Sall restriction enzymes, were ligated to linear pDCM2 (Korean Publication No. 10-2020-0136813) using T4 ligase (New England Biolabs, Beverly, MA). The resulting vector was named pDCM2-ΔNCgl1292down.

PCR was performed based on pDCM2-ΔNCgl2195down::Pcj7_ald1(B.li) constructed in Example 1-1 above as a template using a primer pair of SEQ ID NOS: 32 and 33. PCR was performed under conditions of denaturation at 95°C for 5 minutes, followed by 30 cycles of denaturation at 95°C for 30 seconds, annealing at 55°C for 30 seconds, and polymerization at 72°C for 30 seconds, and then polymerization at 72°C for 5 minutes. As a result, a 1,598 bp of Pcj7_ald1(B.li) polynucleotide was obtained. The fragment, which was cleaved with Notl and Xhol restriction enzymes, was ligated to linear pDCM2-ΔNCgl1292down using T4 ligase (New England Biolabs, Beverly, MA). The resulting vector was named pDCM2-ΔNCgl1292down::Pcj7_ald1(B.li).

**[Table 4]**

| SEQ ID NO: | Name | Sequence |
|---|---|---|
| 28 | N1292dw_AF | gagctcggtacccggggatccGTTTTCTACAATAAAT TGAGTTAG |
| 29 | N1292dw_AR | CAACCGGACGCATGGCActcgaggcggccgcGA AAAAGGCTTTCG |
| 30 | N1292dw_BF | CGAAAGCCTTTTTCgcggccgcctcgagTGCCAT GCGTCCGGTTG |
| 31 | N1292dw_BR | catgcctgcaggtcgacCGCTCATTGGGTACGG |
| 32 | Pcj7_ald1(B.li)_F_ N1292 | GAAAGCCTTTTTCgcggccgcAGAAACATCCCA GCGCTAC |
| 33 | ald1(B.li)_R_N129 2 | CTTCACAACCGGACGCATGGCActcgagTTAA AGCAGAATGG |

### Example 2-2: Construction of Microorganisms with Additional Copies of Exogenous Alanine Dehydrogenase Gene

The pDCM2-ΔNCgl1292down::Pcj7_ald1(B.li) vector constructed in Example 2-1 was transformed into each of CJ0183 and CJ0184, which are the parent strains, by electroporation, and then the strains, in which the variant gene and the vector were inserted into the chromosome, were selected as primary candidates in a selection medium containing 25 mg/L of kanamycin. Thereafter, one copy of ald1(B.li) was additionally introduced into the endogenous ΔNCgl1292down intergenic region on the chromosome through a secondary crossover process using homology between the gene existing on the chromosome and the gene being inserted through the vector. Final strains, from which the vector containing a kanamycin resistance gene was removed, were obtained. The final strains were primarily examined through PCR using a primer pair of SEQ ID NO: 34 and SEQ ID NO: 35, and were finally confirmed through sequencing. The thus-obtained strains introduced with additional copies of Pcj7_ald1(B.li) were named CJ0242 and CJ0229, respectively. That is, CJ0242 is a strain in which two copies of B.li ald1 were added relative to ATCC13869, and CJ0229 is a strain in which one copy of B.li ald1 and 1 copy of B.li ald2 were added relative to ATCC13869.

**[Table 5]**

| SEQ ID NO: | Name | Sequence |
|---|---|---|
| 34 | N 1292down CF | CAGTCAATAGGTAGTCCCGATCCCAAG |
| 35 | N 1292down CR | GAACTAACGAAGAAGGACGCCTAAGAGA C |

### Example 2-3: Evaluation of L-alanine-Producing Ability of L-alanine-Producing Microorganisms with Additional Copies of Exogenous Alanine Dehydrogenase Gene

In order to confirm the L-alanine productivity of the *Corynebacterium glutamicum* CJ0242 and CJ0229 constructed in Example 2-2 above, the strains were cultured as follows:
The *Corynebacterium glutamicum* ATCC13869 and the five types of variant strains were inoculated into a 250-mL corner-baffled flask containing 25 mL of the following seed medium and was cultured with shaking at 200 rpm at 30°C for 20 hours to obtain a seed culture solution. Thereafter, 1 mL of the seed culture solution was inoculated into a 250-mL corner-baffled flask containing 24 mL of the following production medium and was cultured with shaking at 200 rpm at 30°C for 48 hours to prepare alanine.

After completion of the culture, the production of L-alanine was measured using liquid high-performance chromatography, and the production of L-alanine in the culture solution for each strain tested was measured. The compositions of the seed medium and production medium are as follows, and the L-alanine concentrations in the culture solution for each strain tested are shown in Table 6 below.

**[Table 6]**

| | CJ0183 (Parent strain) | CJ0242 | CJ0184 (Parent strain) | CJ0229 |
|---|---|---|---|---|
| L-alanine Concentratio n (g/L) | 38.2 | 40.8 | 37.4 | 41.5 |

As a result, as shown in Table 6 above, the parent strain CJ0183 produced L-alanine at a concentration of 38.2 g/L, but the CJ0242 strain, into which one additional copy was introduced into the exogenous alanine dehydrogenase-introduced strain according to the present disclosure, produced L-alanine at a concentration of 40.8 g/L, confirming that the L-alanine productivity was increased by 107% compared to the parent strain. Additionally, the parent strain CJ0184 produced L-alanine at a concentration of 37.4 g/L, and the CJ0229 strain, into which one additional copy was introduced into the exogenous alanine dehydrogenase-introduced strain according to the present disclosure, produced L-alanine at a concentration of 41.5 g/L, confirming that the L-alanine productivity was increased by 111% compared to the parent strain.

Accordingly, it was confirmed that L-alanine could be produced at high concentrations by introducing additional copies into the microorganism of the genus *Corynebacterium* introduced with the exogenous alanine dehydrogenase of the present disclosure.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A microorganism of the genus *Corynebacterium* having an L-alanine-producing ability, into which the activity of alanine dehydrogenase is introduced,
wherein the alanine dehydrogenase is derived from *Bacillus licheniformis, Bacillus amyloliquefaciens,* or *Laceyella sacchari.*

2. The microorganism of claim 1, wherein the alanine dehydrogenase is encoded by the *ald1, ald2,* or *alaD* gene.

3. The microorganism of claim 1, wherein the alanine dehydrogenase comprises an amino acid sequence of SEQ ID NO: 1, SEQ ID NO: 3, SEQ ID NO: 5, or SEQ ID NO: 7.

4. The microorganism of claim 1, wherein the gene encoding the alanine dehydrogenase comprises a nucleotide sequence of SEQ ID NO: 2, SEQ ID NO: 4, SEQ ID NO: 6, or SEQ ID NO: 8.

5. The microorganism of claim 1, wherein the microorganism of the genus *Corynebacterium* is *Corynebacterium glutamicum.*

6. The microorganism of any one of claims 1 to 5, wherein the microorganism of the genus *Corynebacterium* has an increased L-alanine-producing ability compared to a non-modified microorganism.

7. A method for producing L-alanine, comprising: culturing a microorganism of the genus *Corynebacterium* having an L-alanine-producing ability, into which the activity of alanine dehydrogenase is introduced, in a medium,
wherein the alanine dehydrogenase is derived from *Bacillus licheniformis, Bacillus amyloliquefaciens,* or *Laceyella sacchari.*

8. The method of claim 7, further comprising recovering a target substance from the cultured microorganism, a culture product of the microorganism, a fermented product of the microorganism, or the culture medium.

9. A composition for producing L-alanine, comprising a microorganism of the genus *Corynebacterium* having an L-alanine-producing ability, into which the activity of alanine dehydrogenase is introduced, a culture product of the microorganism, a fermented product of the microorganism, or a combination of two or more thereof,
wherein the alanine dehydrogenase is derived from *Bacillus licheniformis, Bacillus amyloliquefaciens,* or *Laceyella sacchari.*

10. Use of a microorganism of the genus *Corynebacterium,* into which the activity of alanine dehydrogenase is introduced, for the production of L-alanine,
wherein the alanine dehydrogenase is derived from *Bacillus licheniformis, Bacillus amyloliquefaciens,* or *Laceyella sacchari.*
